(19) Europäisches Patentamt
European Patent Office
Office européen des brevets

(11) Numéro de publication: **0 463 947 B1**

# (12) FASCICULE DE BREVET EUROPEEN

(45) Date de publication du fascicule du brevet: **25.10.95**

(51) Int. Cl.[6]: **C07C 323/54**, C07C 323/12, C07D 317/44, C07C 317/18, C08F 128/02

(21) Numéro de dépôt: **91401685.2**

(22) Date de dépôt: **21.06.91**

(54) **Procédé de préparation de composés acryliques soufrés.**

(30) Priorité: **27.06.90 FR 9008109**

(43) Date de publication de la demande:
**02.01.92 Bulletin 92/01**

(45) Mention de la délivrance du brevet:
**25.10.95 Bulletin 95/43**

(84) Etats contractants désignés:
**AT BE CH DE DK ES FR GB GR IT LI LU NL SE**

(56) Documents cités:
**SU-A- 432 128**

**TETRAHEDRON LETTERS, vol. 25, no. 26, 1984, pages 2787-2790, Pergamon Press, Ltd, GB; D.H.R. BARTON et al.: "Carbethoxyallylation using radical chemistry"**

**CHEMICAL ABSTRACTS, vol. 84, 31 mai 1976, page 16, résumé no. 151191y, Columbus, Ohio, US; G. N. SHVAREVA et al, "Oxidative thermal stability of copolymers of methyl methacrylate and thioalkyl acrylates"**

(73) Titulaire: **ELF ATOCHEM S.A.**
**4 & 8, Cours Michelet**
**La Défense 10**
**F-92800 Puteaux (FR)**

(72) Inventeur: **Cerf, Martine**
**2, rue C. Psitser**
**F-57000 Metz (FR)**
Inventeur: **Mieloszynski, Jean-Luc**
**286, Rue de Pont à Mousson**
**F-57158 Montigny les Metz (FR)**
Inventeur: **Paquer, Daniel**
**2, Rue d'Anvers**
**F-54500 Vandoeuvre (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention se rapporte à un procédé de préparation de composés acryliques soufrés.

On connaît actuellement un petit nombre d'acrylates et méthacrylates soufrés obtenus par réaction d'alcools soufrés avec soit un (méth)acrylate d'alkyle soit un chlorure de (méth)acryloyle. Ainsi des (méth)-acrylates de formule :

$$H_2C = C(R^1) - C(=O) - O - (CH_2)_n - SO_m - R^2 \qquad (I)$$

dans laquelle $R^1$ est l'hydrogène ou bien un radical méthyle, n est un nombre entier de 1 à 4, $R^2$ est un radical alkyle ayant de 1 à 4 atomes de carbone et m = 0 ou 2 sont connus par le brevet US-A-3 278 500. Chemical Abstracts 86, 189153s. Des (méth)acrylates de formule

$$H_2C = C(R^1) - C(=O) - (OCH_2CH_2)_n - S - (CH_2)_m - R^2 \qquad (II)$$

dans laquelle n est un nombre entier de 1 à 3, $R^1$ est l'hydrogène ou bien un radical méthyle, $R^2$ est un radical phényle ou naphtyle et m = 0 ou 1 sont connus par le brevet DE-A-3 321 501. Chemical Abstracts 84, 151191y pour sa part décrit des (méth)acrylates de formule (I) dans laquelle n = 2, m = 0 et $R^2$ peut être un radical alkyle tertiaire ayant de 4 à 8 atomes de carbone.

On connaît également par le brevet soviétique n° 432 128 un procédé d'obtention de (méth)acrylates de formule (I) dans laquelle $R^1$ est l'hydrogène ou bien un radical méthyle, n = 1, m = 0 et $R^2$ est choisi parmi les radicaux alkyle et les radicaux alcényle, par réaction d'un sel de potassium de l'acide acrylique ou méthacrylique avec un composé de formule $R^2SCH_2Cl$, la réaction étant effectuée dans le toluène pendant 2 heures à une température de 110° à 115°C et en présence d'un halogénure de tétraalkylammonium.

Cette réaction connue, qui permet d'obtenir des composés de formule (I) avec un rendement de 63 à 85%, présente l'inconvénient de nécessiter une température élevée et un solvant organique, avec tous les problèmes énergétiques et d'environnement que cela suppose notamment en ce qui concerne le recyclage et la purification du solvant pour permettre une utilisation efficace tout en évitant son rejet dans la nature. D'autre part cette réaction n'a pas pu être transposée avec de bons rendements à la synthèse d'autres (méth)acrylates soufrés.

Un problème que la présente invention vise à résoudre consiste à mettre au point un procédé permettant l'obtention d'acrylates et méthacrylates soufrés à température modérée avec des rendements élevés et, dans la mesure du possible, en limitant le recours à des solvants organiques.

Des acrylates de polythiaalkyle contenant de 3 à 12 atomes de carbone et contenant deux atomes de soufre séparés par au moins un atome de carbone sont connus par le brevet US-A-3 536 677. Ces composés, tels que le bis(2-thiobutyl)méthyl acrylate de formule

$$CH_2 = CH - C(=O) - O - CH(CH_2SC_2H_5)_2 \qquad (III)$$

sont utiles dans la composition de copolymères pour émulsions photographiques à base d'halogénure d'argent. Ils sont obtenus par réaction dans l'éther d'un polythiaalcool sur le chlorure d'acryloyle en présence de triéthylamine. Comme dans le cas des composés de formule (I), l'objectif de la présente invention consiste à améliorer les rendements et si possible à s'affranchir de l'emploi de solvants organiques.

Des alkylsulfinyl(méth)-acrylates de formule :

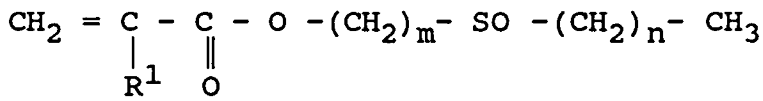

(IV)

dans laquelle $R^1$ est l'hydrogène ou bien un groupe méthyle, m = 0 à 18 et n = 0 à 1 sont également connus par le brevet DE-A-2 434 550. Ces composés sont obtenus par oxydation d'alkylthioacrylates en présence de $NaIO_4$.

Un autre objectif de la présente invention vise à proposer un procédé simple et efficace pour la synthèse de sulfones et sulfoxydes afin d'élargir la gamme des composés disponibles.

On connaît par le brevet FR-A-2 517 201 des disulfures et trisulfures de formule $R^2S_xR^1$ dans laquelle x = 2 ou 3 et $R_1$ signifie un groupe hydroxyalkyle, hydroxycycloalkyle, alcoxyalkyle, alcoxycycloalkyle, aminoalkyle, aminocycloalkyle ou époxyalkyle, $R_2$ signifie un groupe alkyle ou cycloalkyle ou possède l'une des significations données pour $R_1$.

La présente invention est basée sur la découverte que les divers problèmes et objectifs évoqués ci-dessus au sujet des (méth)acrylates soufrés de formules (I) à (IV), c'est-à-dire de type sulfures, sulfones et sulfoxydes, peuvent être résolus par un nouveau procédé consistant en la préparation d'un (méth)acrylate de formule générale :

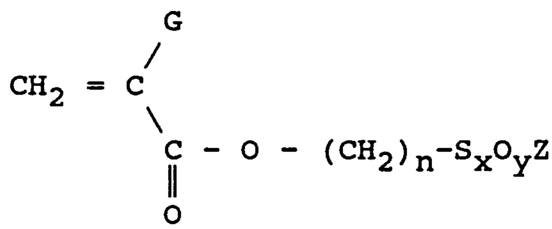

(VI)

dans laquelle :

- G désigne un atome d'hydrogène ou un radical méthyle ;
- n est un nombre entier compris entre 1 et 12 inclusivement,
- x vaut 1 ou 2,
- y vaut 0, 1 ou 2, y ne pouvant représenter 1 ou 2 que lorsque x = 1, et
- Z est un radical alkyle, cycloalkyle, alcényle, aryle, alkylaryle, arylalkyle ou $\beta$-thiénylméthyle, Z étant porteur le cas échéant d'une fonction ester,

à partir d'un (méth)acrylate de formule générale :

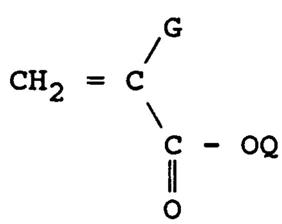

(VII)

dans laquelle G est tel que défini ci-dessus et Q est un métal alcalin,

par réaction dudit (méth)acrylate de formule (VII) avec un composé soufré de formule $ZS(CH_2)_nX$ dans laquelle x, n et Z ont les significations indiquées précédemment et X est un atome d'halogène, ladite réaction étant effectuée soit en milieu au moins partiellement aqueux à une température de 50 °C à 100 °C soit dans un solvant organique dont la température d'ébullition ne dépasse pas 100 °C, en présence d'au moins un inhibiteur de polymérisation et d'au moins un agent de transfert de phase et conduisant à un (méth) acrylate de formule (VI-a) dans laquelle y = 0 et x = 1 ou 2, auquel cas, si x = 1, cette première réaction peut être suivie par une réaction d'oxydation au moyen d'au moins un agent oxydant à une température généralement comprise entre 10 °C et 60 °C environ afin d'obtenir un (méth)acrylate de

formule (VI) dans laquelle y vaut 1 ou 2.

Le procédé qui vient d'être décrit dans son expression la plus générale présente les avantages suivants :

- absence, ou tout au moins minimisation de la présence, de solvant organique,
- élimination facile de l'halogénure alcalin QX produit par la réaction, par simple décantation dans la phase aqueuse,
- récupération aisée de l'acrylate ou du méthacrylate de formule (VI) par simple lavage à l'eau de la phase organique, et
- rendements de réaction élevés, quelles que soient les valeurs de n, x et y.

La réaction du (méth)acrylate de formule (VII) et du composé soufré est effectuée en utilisant l'eau comme solvant, auquel cas la température réactionnelle est de préférence comprise entre 70 °C environ et 100 °C, ou bien en utilisant comme solvant un mélange d'eau et d'un solvant organique tel que le chloroforme, le chlorure de méthylène, le tétrachlorure de carbone, le benzène ou le toluène. Le recours à l'eau comme seul solvant sera tout particulièrement préféré pour la préparation d'un (méth)acrylate de formule (VI), la quantité d'eau utilisée pouvant alors être très faible, c'est-à-dire tout juste suffisante pour la dilution du (méth)acrylate alcalin. A titre d'exemple, et en fonction de la nature du métal alcalin, la quantité d'eau peut être aussi faible que 5 moles pour une mole de (méth)acrylate alcalin. Dans le cas où un solvant organique est utilisé, la température réactionnelle dépendra de la nature de ce solvant et de sa proportion vis-à-vis de l'eau. Généralement on préfèrera choisir comme température réactionnelle la température de reflux du mélange d'eau et de solvant organique, par exemple 57 °C dans le cas d'un mélange équipondéral d'eau et de chloroforme.

La réaction du (méth)acrylate de formule (VII) et du composé soufré est effectuée en présence d'au moins un composé capable d'inhiber la polymérisation du (méth)acrylate de formule (VII) et/ou du (méth)-acrylate de formule (VI), utilisé par exemple à raison de 0,05% à 0,5% environ en poids sur la base du poids du (méth)acrylate de formule (VII). Comme exemples d'inhibiteurs de polymérisation utilisables, on peut citer notamment la phénothiazine, l'éther méthylique de l'hydroquinone, la N,N-diéthylhydroxylamine, le nitrobenzène, le di-tertiobutylcatéchol, l'hydroquinone, le p-anilinophénol, le phosphite de di-(2-éthylhexyl)-octylphényle, le bleu de méthylène et leurs mélanges en toutes proportions.

La réaction du (méth)acrylate de formule (VII) et du composé soufré est effectuée en présence d'au moins un agent de transfert de phase, utilisé par exemple à raison de 5% à 30% environ en moles par rapport au (méth)acrylate de formule (VII). Comme exemples d'agents de transfert de phase utilisables conformément au procédé selon l'invention on peut citer notamment :

- les sels d'ammonium quaternaires tels que le chlorure de triméthylbenzylammonium, le chlorure de triéthylbenzylammonium, le chlorure de tétraméthylammonium, le bromure de tétraméthylammonium, le bromure de tétrabutylammonium, l'iodure de tétraéthylammonium, le chlorure de tricaprylméthylammonium et l'hydrogénosulfate de tétrabutylammonium,
- les sels de phosphonium quaternaires tels que le bromure de tétrabutylphosphonium, l'iodure de triphényliodophosphonium, etc.,
- les sels d'arsénium quaternaires,
- les éthers de polyéthylène glycols,
- les aminoéthers,
- les complexants macrohétérocycliques de la famille des cryptants aprotiques tels que ceux décrits dans le document FR-A-2 398 079, et
- les complexants macrocycliques non azotés, tels que des polyéthers cycliques (encore dénommés éthers-couronne) et des polythioéthers cycliques, tels que notamment des polyéthers macrocycliques dont l'anneau macrocyclique contient au moins 14 atomes de carbone et d'oxygène, chaque atome d'oxygène de l'anneau étarit séparé des autres atomes d'oxygène de l'anneau par deux ou bien trois atomes de carbone ; de tels polyéthers macrocycliques ont déjà été décrits dans le brevet US-A-3 687 978.

Des exemples plus précis sont notamment :

- le 1,4,7,10,13,16-hexaoxacyclooctadécane,
- le 2,2,11,12-dibenzo-1,4,7,10,13,16-hexaoxacyclooctadéca-1,11-diène,
- le 2,3,12,13-dibenzo-1,4,11,14-tétraoxacycloeicosa-2,12-diène,
- le 2,3,12,13,22,23-tribenzo-1,4,11,14,21,24-hexaoxacyclotriaconta-2,12,22-triène,
- le 2,2,7,7,12,12,17,17-octaméthyl-21,22,23,24-tétraoxaquaterène,
- le 2,3-benzo-1,4,7,10,13-pentaoxacyclopentadéca-2-ène,
- le 2,3-(4'-t-butyl)-1,4,7,10,13,16-hexaoxacyclooctadéca-2-ène,
- le 2,3,9,10-dibenzo-1,4,8,11-tétraoxacyclotétradéca-2,9-diène,

- le 2,3,32,33-dibenzo-1,4,7,10,13,16,19,22,25,28,31,34,37,40,43,46,49,52,55,58-eicosaoxacyclohexaconta-2,32-diène,
- le 2,3,16,17-dibenzo-1,4,15,18-tétraoxacyclooctacosa-2,16-diène,
- le 2,6,13,17-tétraoxatricyclo[16.4.O.$O^{7,12}$]-docosane,
- le 2,5,8,15,18,21-hexaoxatricyclo[20.4.O.$O^{9,14}$]-hexacosane,
- le 2,5,12,15,18-pentaoxatricyclo[17.4.O.$O^{6,11}$]-tricosane,
- le 2,6,13,16,19-pentaoxatricyclo[18.4.O.$O^{7,12}$]-tétracosane,
- le 9,10-benzo-2,5,8,11,14,17-hexaoxabicyclo-[16.4.O]-docosa-9-ène,
- le 2,3,9,10-dibenzo-1,4,8,11,14,16-hexaoxacyclooctadéca-2,9-diène,
- le 2,3,11,12-dibenzo-1,4,7,10,13,16,18-heptaoxacycloeicosa-2,11-diène,
- le 2,3,13,14-dibenzo-8-pentaméthylène-1,4,7,9,12,15,18-heptaoxacycloeicosa-2,13-diène,
- le 2,3,13,14-dibenzo-1,4,7,9,12,15,18,20-octa-oxacyclodocosa-2,13-diène,et
- le 2,4-(1',8'-naphtylène)-1,5,8,11,14-penta-oxacyclohexadéca-2-ène.

Q est un métal alcalin qui peut être choisi parmi le lithium, le sodium et le potassium, ces deux derniers métaux étant préférés. L'halogène X du composé de formule $ZS_x(CH_2)_nX$ peut être choisi parmi le fluor, le chlore, le brome et l'iode, le chlore étant généralement préféré.

Le (méth)acrylate de formule (VII) et le composé soufré sont généralement, dans le procédé selon l'invention, utilisés en proportions telles que le rapport molaire

$$\frac{\text{(méth)acrylate (VII)}}{\text{composé soufré}}$$

soit au moins égal à 1 et de préférence compris entre 1 et 1,6 environ. La durée de la réaction est généralement comprise entre 30 minutes et 20 heures environ.

La réaction du (méth)acrylate de formule (VII) et du composé soufré conduit à un (méth)acrylate de formule (VI-a) dans laquelle G est l'hydrogène ou un radical méthyle et y = 0, qu'il reste à oxyder dans une seconde étape pour atteindre le (méth)acrylate de formule (VI) dans laquelle y = 1 ou bien y = 2. Cette réaction d'oxydation a lieu à température modérée, le plus souvent inférieure à celle de la première étape, et en présence d'un agent oxydant qui peut être :

- l'eau oxygénée, de préférence à une concentration comprise entre 5% et 50% environ,
- les peracides organiques, tels que l'acide peracétique et l'acide performique,
- l'oxygène, l'air ou le diméthylsulfoxyde.

Cet agent oxydant est utilisé en proportion telle que le rapport molaire

$$\frac{\text{agent oxydant}}{\text{(méth)acrylate (VI-a)}}$$

soit au moins égal à 1 pour atteindre un sulfoxyde (y = 1), à 2 pour atteindre une sulfone (y = 2). La durée de la réaction est généralement comprise entre 3 et 90 heures environ, selon la température réactionnelle choisie.

A l'issue de la première étape du procédé selon l'invention (obtention d'un (méth)acrylate de formule (VI-a avec y = 0) ou, le cas échéant, de la seconde étape (obtention de sulfoxyde ou sulfone), le produit formé par la réaction peut être aisément isolé du milieu réactionnel par l'une des méthodes suivantes :

- cas d'un sulfure (y = 0) obtenu en utilisant l'eau comme solvant : la phase organique est recueillie, le cas échéant avec l'aide d'une extraction par un solvant organique tel que le dichlorométhane, lavée à l'eau puis séchée. Dans le cas d'une extraction, le (méth)acrylate de formule (VI-a) est obtenu par évaporation du solvant organique,
- cas d'un sulfure (y = 0) obtenu en utilisant comme solvant un mélange d'eau et d'un solvant organique : le mélange réactionnel est décanté et la phase organique est lavée à l'eau puis séchée, et le (méth)acrylate de formule (VI-a) est obtenu par évaporation du solvant organique,
- cas d'un sulfoxyde (y = 1) ou d'une sulfone (y = 2) : le mélange réactionnel est extrait au moyen d'un solvant organique tel que le chloroforme puis, après séchage de la phase organique, le (méth)-acrylate de formule (VI) est obtenu par évaporation du solvant organique.

Certains des problèmes et objectifs évoqués précédemment au sujet des (méth)acrylates soufrés de formules (I) à (IV) peuvent également être résolus par un procédé en tous lieux identique au premier procédé décrit précédemment, à l'exception du fait que la réaction entre le (méth)acrylate de formule (VII) et le composé soufré est effectué dans un solvant organique dont la température d'ébullition ne dépasse pas 100 ° C.

Ce procédé, quoique moins avantageux que le précédent sur le plan du respect de l'environnement puisqu'il ne s'affranchit pas de l'emploi d'un solvant organique, présente toutefois, vis-à-vis des procédés utilisant des solvants aromatiques (toluène) connus notamment par le brevet soviétique n° 432 128, les avantages suivants :

- obtention de rendements de réaction élevés, quelles que soient les valeurs de n, x et y, notamment dans le cas de certaines synthèses pour lesquelles les rendements obtenus dans le toluène sont médiocres,
- diminution de la température réactionnelle jusqu'à une valeur ne dépassant pas 100 ° C,
- élimination facile de l'halogénure alcalin QX produit par la réaction, par simple filtration.

Dans ce second procédé, la température réactionnelle est généralement la température de reflux du solvant organique choisi et dépend donc de la nature de celui-ci. A titre d'exemple on utilisera avantageusement l'acétonitrile en opérant à la température de 80 ° C environ. L'inhibiteur de polymérisation et l'agent de transfert de phase sont choisis de la même manière que dans le premier procédé décrit précédemment. La réaction d'oxydation permettant de transformer un sulfure (y = 0) en sulfoxyde (y = 1) ou sulfone (y = 2) est également conduite comme décrit précédemment.

Plusieurs des composés soufrés avec lesquels on fait réagir le (méth)acrylate de formule (VII) dans les procédés précédents sont déjà connus. Ainsi le composé $(C_2H_5SCH_2)_2CHOH$ est connu par le brevet US-A-3 536 677, le composé $CH_3S(CH_2)_2OH$ par le brevet US-A-3 278 500, les composés $C_6H_5S(CH_2CH_2O)_nH$(n = 1 à 3) par le brevet DE-A-3 321 501 et les composés de formule $R^1SCH_2OH$, dans laquelle $R^1$ est un radical alkyle ou phényle, sont connus par Chemical Abstracts 86, 189153. Le composé de formule

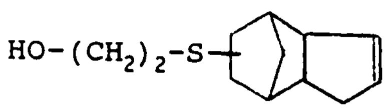

est connu par Chemical Abstracts 109, 55419. Par contre un certain nombre de ces composés soufrés n'ont jamais été décrits jusqu'à présent. Ainsi le composé $(CH_3)_3CS(CH_2)_{11}OH$ est préparé par réaction de l'hydroxy-11 undécène-1 sur le tertiobutylmercaptan en présence d'au moins un initiateur de radicaux libres tel que l'azo-bis-isobutyronitrile, de préférence à la température de reflux d'un solvant hydrocarboné tel que le cyclohexane, le méthylcyclohexane ou l'heptane, le rapport molaire du mercaptan au composé alcénique étant compris entre 1 et 1,3 environ. La même méthode de préparation, appliquée à la réaction du mercapto-2 éthanol sur le dicyclopentadiène, permet d'atteindre un rendement trois fois plus élevé que celui obtenu selon la méthode de la demande de brevet JP-A-62/283 958 (C.A. 109, 55419). Après refroidissement la solution est lavée par de l'eau alcaline, séchée, puis le solvant est évaporé pour récupérer le produit de la réaction.

Les alcools disoufrés de formule $(CH_3)_3CS_2(CH_2)_nOH$ dans laquelle n est compris entre 2 et 12 sont préparés par réaction de tertiobutylmercaptan sur un chloroalcool de formule $Cl-(CH_2)_n-OH$ en présence de soufre, la réaction étant effectuée au reflux d'un alcool léger tel que l'éthanol, en milieu alcalin et avec des rapports molaires de sensiblement 1 mole de mercaptan pour 1 mole de chloroalcool et 1 mole de soufre. Les rendements observés pour cette réaction sont d'autant plus élevés que n est plus grand. Lorsque n = 2, on préférera la méthode de synthèse en trois étapes consistant à faire réagir le chlorure de sulfényle sur le méthanol, par exemple à température ambiante dans l'éther diéthylique, puis à faire réagir le composé $ClSCO_2CH_3$ obtenu avec le mercapto-2 éthanol, par exemple dans le méthanol à 0 ° C, et enfin à faire réagir le disulfure $CH_3CO_2S_2(CH_2)_2OH$ obtenu avec le tertiobutylmercaptan en présence d'une quantité catalytique de triéthylamine, par exemple dans le méthanol à une température de 10 ° à 40 ° C environ. Cette dernière méthode, malgré le nombre d'étapes, permet d'obtenir l'alcool disoufré $(CH_3)_3CS_2(CH_2)_2OH$ avec un rendement plus de deux fois supérieur à celui de la première méthode décrite précédemment.

Le chlorure soufré de formule

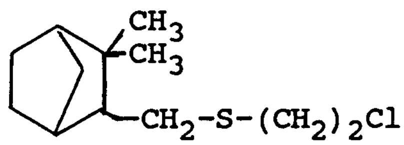

est obtenu par réaction du chlorure de thionyle avec l'alcool soufré

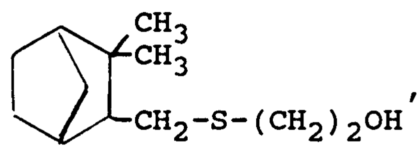

par exemple au reflux d'un solvant tel que le chloroforme, puis isolé par évaporation du chloroforme.

On pense que sont nouveaux les composés acryliques soufrés, susceptibles d'être préparés par le procédé décrit précédemment, choisis parmi ceux de formule :

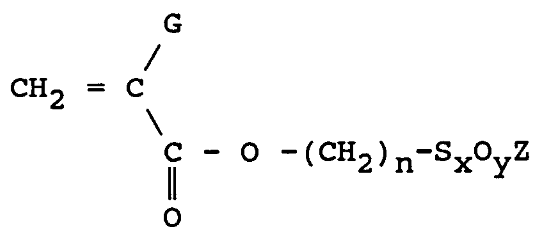

(IX)

dans laquelle :

- G est tel que défini ci-dessus et
- soit n est un nombre entier de 1 à 12, x est un nombre entier de 1 à 3 et y est un nombre entier de 0 à 2, auquel cas Z est choisi parmi les radicaux alkyles, alcényles, aryles, alkylaryles et arylalkyles porteurs d'une fonction ester ou parmi les radicaux cycloalkyles et hétérocycliques porteurs le cas échéant d'une fonction ester,
- soit n est un nombre entier de 1 à 12, X est égal à 1 et y est égal à 1, auquel cas Z est choisi parmi les radicaux aryles, alcényles, alkylaryles et arylalkyles,
- soit n est un nombre entier de 5 à 12, X est égal à 1 et y est égal à 0 ou 2, auquel cas Z est choisi parmi les radicaux alkyles, alcényles, aryles, alkylaryles et arylalkyles,
- soit n est un nombre entier de 1 à 12, X est égal à 2 ou 3 et y est un nombre entier de 0 à 2, auquel cas Z est choisi parmi les radicaux alkyles, alcényles, aryles, alkylaryles et arylalkyles.

Un grand nombre de composés entrant dans la définition ci-dessus sont décrits et caractérisés analytiquement dans les exemples ci-après.

Ces composés acryliques et méthacryliques décrits précédemment sont applicables à la constitution de nouveaux polymères et copolymères. Plus précisément la présente invention concerne des polymères et copolymères comprenant au moins un motif dérivé d'au moins un composé acrylique ou méthacrylique décrit précédemment ou obtenu par le procédé décrit précédemment. De tels (co)polymères peuvent en outre comprendre au moins un motif dérivé d'au moins un comonomère copolymérisable avec ledit composé acrylique soufré, tel que par exemple :

- un acrylate ou méthacrylate d'alkyle dont le groupe alkyle linéaire ou ramifié, le cas échéant substitué, par exemple par au moins un atome d'halogène comme le chlore ou le fluor et/ou par au moins un groupe hydroxyle, possède de 1 à 20 atomes de carbone,
- un acrylate ou méthacrylate d'aryle tel que le méthacrylate de benzyle,
- un hydrocarbure vinylaromatique tel que le styrène, le vinyltoluène, l'alphaméthylstyrène, le méthyl-4 styrène, le méthyl-3 styrène, le méthoxy-4 styrène, l'hydroxyméthyl-2 styrène, l'éthyl-4 styrène, l'éthoxy-4 styrène, le diméthyl-3,4 styrène, le chloro-2 styrène, le chloro-3 styrène, le chloro-4 méthyl-3 styrène, le tert.-butyl-3 styrène, le dichloro-2,4 styrène, le dichloro-2,6 styrène et le vinyl-1 naphtalène,

- un nitrile insaturé tel que l'acrylonitrile ou le méthacrylonitrile.
- une maléimide N-substituée telle que la N-éthylmaléimide, la N-isopropylmaléimide, la N-n-butylmaléimide, la N-isobutylmaléimide, la N-terbutylmaléimide, la N-n-octylmaléimide, la N-cyclohexylmaléimide, la N-benzylmaléimide et la N-phénylmaléimide,
- un anhydride d'acide dicarboxylique insaturé tel que l'anhydride maléique, l'anhydride itaconique, l'anhydride citraconique ou l'anhydride tétrahydrophtalique,
- l'acide acrylique ou méthacrylique,
- un acrylate ou méthacrylate de polyol comme les diacrylates et diméthacrylates de l'éthylèneglycol, du propylèneglycol, du 1,3-butanediol, du 1,4-butanediol, du 1,6-hexane-diol, du néopentylglycol, du 1,4-cyclohexane-diol, du 1,4-cyclohexanediméthanol, du 2,2,4-triméthyl-1,3-pentanediol, du 2-éthyl-2-méthyl-1,3-propanediol, du 2,2-diéthyl-1,3-propanediol, du diéthylèneglycol, du dipropylèneglycol, du triéthylèneglycol, du tripropylèneglycol, du tétraéthylèneglycol, du tétrapropylèneglycol, du triméthyloléthane, du triméthylolpropane, du glycérol, du pentaérythritol, les triacrylates et triméthacrylates du triméthyloléthane, du triméthylolpropane, du glycérol, du pentaérythritol, les tétraacrylates et tétraméthacrylates du pentaérythritol, les di(méth)acrylates à hexa(méth)acrylates du dipentaérythritol, les poly(méth)acrylates de polyols mono- ou polyéthoxylés ou mono- ou polypropoxylés tels le triacrylate et le triméthacrylate du triméthylolpropane triéthoxylé, du triméthylolpropane tripropoxylé ; le triacrylate et le triméthacrylate du glycérol tripropoxylé ; le triacrylate, le triméthacrylate, le tétraacrylate et le tétraméthacrylate du pentaérythritol tétraéthoxylé,
- un acrylate ou méthacrylate époxydé choisi parmi le 2-époxyéthylbicyclo[2.2.1]hept-5(6)-yl (méth)-acrylate, l'acrylate d'époxydicyclopentyloxyéthyle ainsi que ceux de formule :

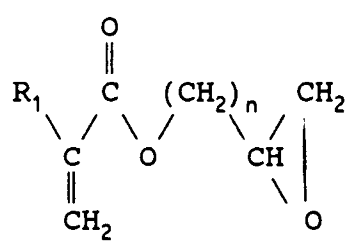

dans laquelle $R_1$ est choisi parmi l'atome d'hydrogène et le radical méthyle, et n est un nombre entier allant de 1 à 16, ceux de formule :

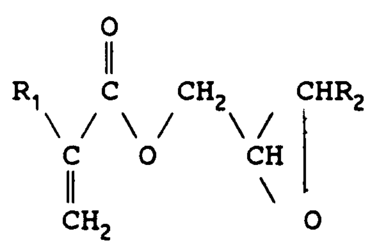

dans laquelle $R_1$ est choisi parmi l'atome d'hydrogène et le radical méthyle, et $R_2$ est choisi parmi les radicaux alkyle ayant de 1 à 12 atomes de carbone et les radicaux aryle ayant de 6 à 12 atomes de carbone, et ceux de formules :

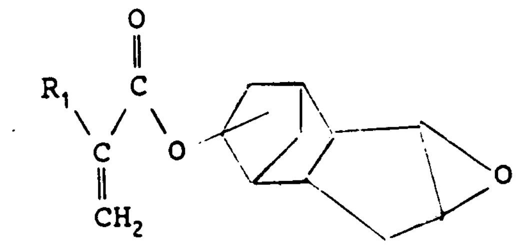

et

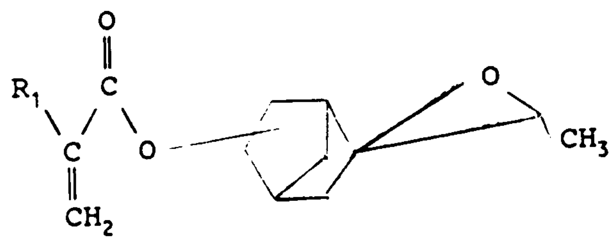

dans lesquelles $R_1$ est choisi parmi l'atome d'hydrogène et le radical méthyle,

- un acrylamide ou méthacrylamide, un acrylate ou méthacrylate de dialkylaminoalkyle et leurs sels quaternaires,
- l'acrylate et le méthacrylate de (norbornyloxy-2)-2 éthyle et de (diméthanodécahydronaphtyloxy-2)-2 éthyle, et
- des oxazolidones acryliques et méthacryliques choisies parmi celles de formule :

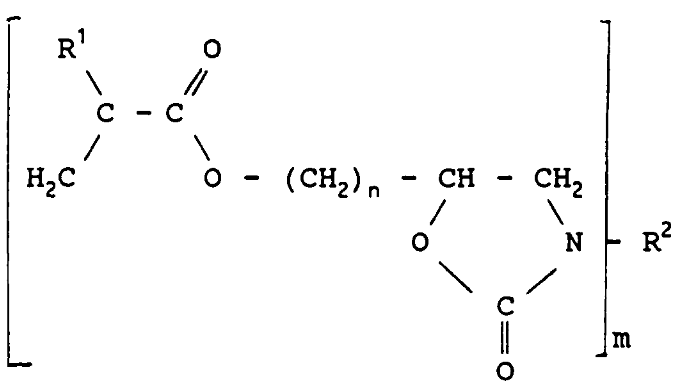

et celles de formule :

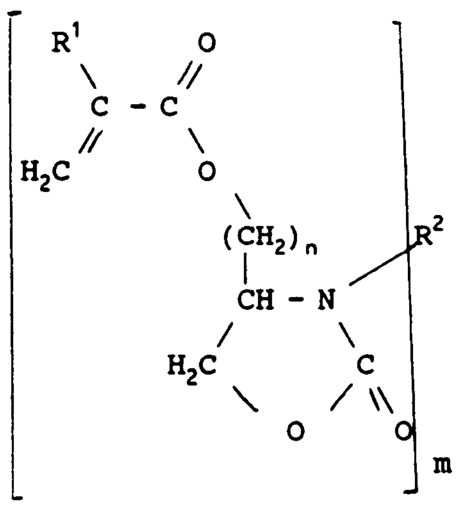

formules dans lesquelles :

- $R^1$ est choisi parmi l'atome d'hydrogène et le radical méthyle,
- n est un nombre entier allant de 1 à 12,
- m est un nombre entier allant de 1 à 3, et
- $R^2$ est un radical hydrocarboné, alkyle linéaire, ramifié ou cyclique ou bien aromatique, possédant de 5 à 12 atomes de carbone,

lesdites oxazolidones pouvant être obtenues par réaction, entre 30 °C et 90 °C, d'un composé portant une fonction (méth)acrylique avec un composé portant au moins une fonction isocyanate,

- des composés acryliques et méthacryliques choisis parmi ceux de formule :

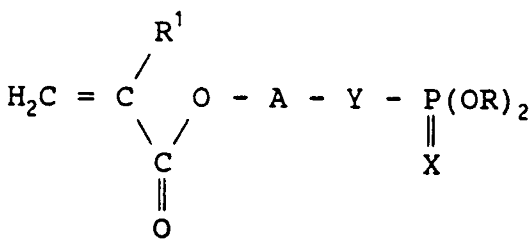

(X)

dans laquelle :

- $R^1$ est choisi parmi l'atome d'hydrogène et le radical méthyle,
- A est choisi parmi les radicaux $(CH_2)_n$ pour lesquels n est un nombre entier de 2 à 12 et le radical $-(CH_2CH_2O)_d-CH_2CH_2-$, d étant un nombre entier allant de 1 à 20,
- X est choisi parmi les atomes de soufre et d'oxygène,
- Y est choisi parmi les atomes de soufre et d'oxygène,

avec la condition que X est un atome de soufre et Y est un atome d'oxygène lorsque A est le radical $-(CH_2CH_2O)_d-CH_2CH_2-$, et

- R est choisi parmi les radicaux alkyle ayant de 1 à 20 atomes de carbone et les groupes $-(CH_2)_pSR^3$ dans lesquels p est un nombre entier allant de 3 à 12 et $R^3$ est un radical alkyle ayant de 1 à 20 atomes de carbone,

ceux de formule :

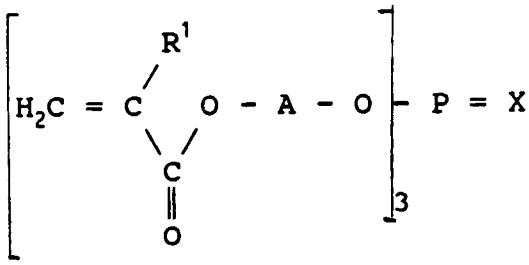

dans laquelle :

- $R^1$ est choisi parmi l'atome d'hydrogène et le radical méthyle,
- A est choisi parmi les radicaux $(CH_2)_n$ pour lesquels n est un nombre entier de 2 à 12 et le radical $-(CH_2CH_2O)_d-CH_2CH_2-$, d étant un nombre entier allant de 1 à 20, et
- X est choisi parmi les atomes de soufre et d'oxygène,

et ceux de formule :

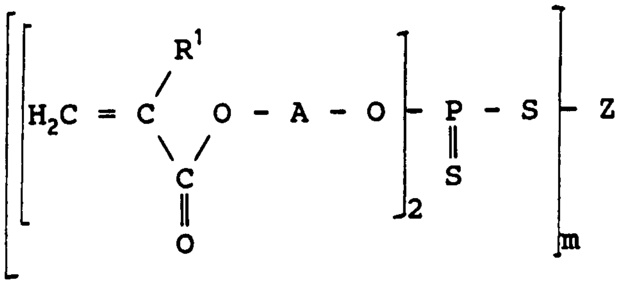

dans laquelle :

- $R^1$ est choisi parmi l'atome d'hydrogène et le radical méthyle,
- A est choisi parmi les radicaux $(CH_2)_n$ pour lesquels n est un nombre entier de 2 à 12,
- m est un nombre entier allant de 1 à 3, et
- Z est choisi parmi l'atome d'hydrogène, les radicaux $R^2QH$, $R^2$ étant un radical alkyle ayant de 2 à 12 atomes de carbone et Q étant choisi parmi les atomes d'oxygène et de soufre, et les atomes des métaux des groupes IA, IIA, IIIA, IB, IIB, VIB, VIIB et VIII de la Classification Périodique, avec la condition que Z est choisi parmi l'atome d'hydrogène et les radicaux $R^2OH$ lorsque m = 1 et que m est la valence de Z lorsque Z est un métal.

De tels composés peuvent être préparés par réaction d'un composé acrylique ou méthacrylique de formule :

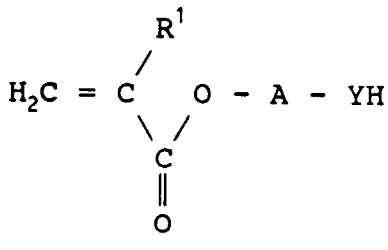

dans laquelle $R^1$, A et Y ont les mêmes significations que dans la formule (X), avec un composé pentavalent du phosphore, celui-ci pouvant être par exemple un composé de formule $PXT_3$ dans laquelle X a la même signification que dans la formule (X) et T désigne un atome d'halogène, ou bien un composé phosphoré de formule :

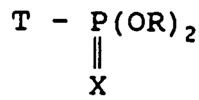

dans laquelle R et X ont les mêmes significations que dans la formule (X) et T désigne un atome d'halogène, ou bien le pentasulfure $P_2S_5$,

- des composés acryliques et méthacryliques choisis parmi ceux de formule :

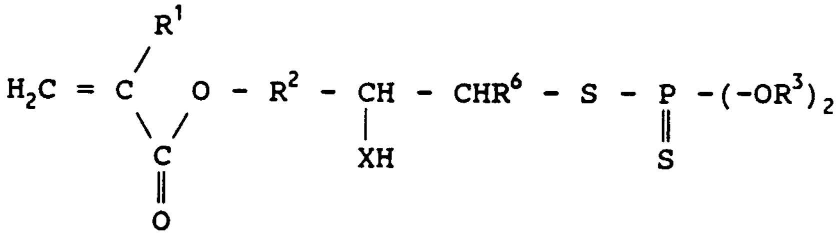 (XI)

et ceux de formule :

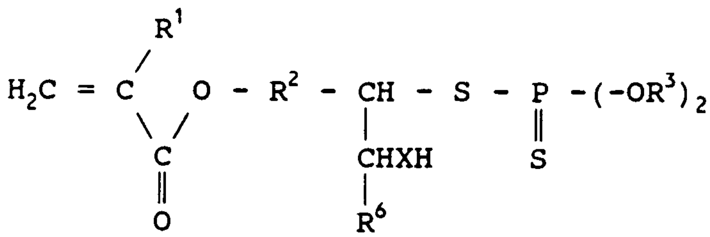

formules dans lesquelles :

- $R^1$ est choisi parmi l'atome d'hydrogène et le radical méthyle,
- X est un hétéroatome choisi parmi l'oxygène et le soufre,
- $R^2$ est choisi parmi les groupes alkylènes linéaires ou ramifiés, cycloalkylènes et hétérocycloalkylènes mono- ou polycycliques, alkylarylènes et arylalkylènes comprenant de 1 à 12 atomes de carbone,
- $R^6$ est choisi parmi l'atome d'hydrogène et les radicaux alkyles et aryles ayant de 1 à 12 atomes de carbone, et
- $R^3$ est choisi parmi les radicaux alkyles et aryles ayant de 1 à 20 atomes de carbone, les groupes $-(CH_2)_pSR^4$ dans lesquels p est un nombre entier allant de 2 à 12 et $R^4$ est un radical alkyle ayant de 1 à 20 atomes de carbone ou bien un groupe cycloalkyle mono- ou polycyclique ayant de 4 à 10 atomes de carbone, chaque cycle dudit groupe comprenant de 4 à 6 chaînons, et les groupes

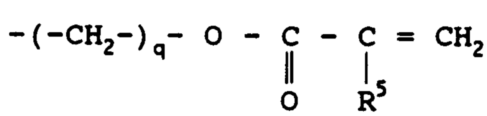

dans lesquels q est un nombre entier allant de 2 à 12 et $R^5$ est choisi parmi l'atome d'hydrogène et le radical méthyle. De tels composés peuvent être préparés par réaction d'un époxyde ou épisulfure acrylique ou méthacrylique de formule :

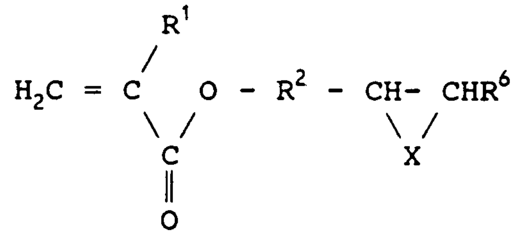

dans laquelle $R^1$, $R^2$, $R^6$ et X ont les mêmes significations que dans la formule (XI), avec un composé thiophosphoré de formule :

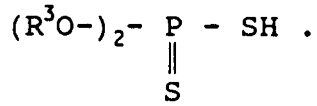

De tels polymères et copolymères sont obtenus en (co)polymérisant au moins un composé acrylique soufré selon l'invention et, le cas échéant, au moins un comonomère copolymérisable, tel que défini précédemment, en présence d'au moins un initiateur de radicaux libres tel qu'un peroxyde, un hydroperoxyde ou un composé diazo. La (co)polymérisation est généralement effectuée à une température comprise entre 50 °C et 120 °C environ et en utilisant l'un des monomères comme solvant. Elle peut également s'effectuer en émulsion dans l'eau, à une température comprise entre 50 °C environ et 100 °C, en présence d'au moins un agent tensio-actif.

Les exemples suivants illustrent la présente invention sans intention limitative.

EXEMPLES 1 à 3

Dans un tricol muni d'un réfrigérant, d'un thermomètre, d'une ampoule à brome et équipé d'une agitation magnétique, on dissout 1 mole de soude dans 400 ml d'éthanol à 95 °C. A 50 °C on ajoute 0,9 mole de mercaptan et on maintient à 50 °C durant 30 minutes. La solution est alors ramenée à température ordinaire afin d'additionner goutte à goutte 0,9 mole du chloroalcool. A la fin de l'addition le mélange est porté à reflux pendant 6h. Après refroidissement le sel formé est éliminé par filtration. Le filtrat est neutralisé par HCl 1N puis la phase organique est extraite avec du dichlorométhane. Les phases organiques sont lavées puis séchées sur du sulfate de sodium. Après élimination du solvant sous pression réduite on récupère un liquide marron qui peut être distillé (sous 1 mmHg) pour donner un liquide incolore.

Dans ces exemples le mercaptan de formule RSH réagit avec le chloroalcool de formule $Cl-(-CH_2)_n-OH$ pour former l'alcool monosoufré de formule $RS-(-CH_2)_n-OH$. Le Tableau I ci-après indique, pour chaque valeur de R et de n, le rendement (Rdt) de la réaction exprimé en pourcentage ainsi que la température d'ébullition T sous 1 mmHg de l'alcool monosoufré formé.

**Tableau I**

| Exemple | R | n | Rdt | T(°C) |
|---|---|---|---|---|
| 1 | tertiobutyle | 5 | 95 | 95 |
| 2 | tertiobutyle | 6 | 95 | 110 |
| 3 | cyclohexyle | 2 | 94 | nd |

nd = non déterminée

EXEMPLE 4

Dans un tricol muni d'un réfrigérant, d'un thermomètre, d'une ampoule à brome et équipé d'une agitation magnétique, on introduit 0,1 mole d'undécène-1-ol-11, 30 ml de cyclohexane et 0,4 g d'azoisobutyronitrile (AIBN).

La solution est ensuite irradiée à l'aide d'une lampe de 200 W (ceci est suffisant pour provoquer le reflux).

On ajoute ensuite goutte à goutte 0,115 mole de tertiobutyl mercaptan. A la fin de l'addition l'irradiation est maintenue pendant 30 mn (on s'assure que la réaction est totale par RMN H1). La solution refroidie à 25 °C est lavée par une solution alcaline (NaOH, 1N) puis à l'eau et enfin séchée sur du sulfate de sodium. Après élimination du solvant sous pression réduite on récupère un liquide jaune.

Le rendement en alcool monosoufré de formule $(CH_3)_3C\text{-}S\text{-}(\text{-}CH_2\text{-})_{11}\text{-}OH$ est de 93%.

EXEMPLES 5 à 11

Dans un tricol muni d'un réfrigérant, d'un thermomètre, d'une ampoule à brome et équipé d'une agitation magnétique, on introduit 40 mmoles d'alcool soufré dans 20 ml de chloroforme sec. On a ajoute goutte à goutte la quantité stoéchiométrique de chlorure de thionyle dilué dans 10 ml de chloroforme. A la moitié de l'addition, le mélange est porté à reflux et le chauffage est poursuivi jusqu'à réaction totale (environ 4h).

Le chloroforme est éliminé sous pression réduite et l'on isole un liquide jaune qui peut être distillé (1 mmHg).

Dans ces exemples l'alcool soufré de formule $RS(CH_2)_2OH$ réagit avec le chlorure de thionyle pour former le chlorure soufré correspondant $RS(CH_2)_2Cl$. Le Tableau II ci-après indique, pour chaque valeur de R, le rendement (Rdt) de la réaction exprimé en pourcentage ainsi que la température d'ébullition T sous 1 mmHg, exprimée en degrés Celsius, du chlorure soufré formé.

Tableau II

| Exemple | R | Rdt | T(°C) |
|---|---|---|---|
| 5 | (3-thiophényl)méthyl | 95 | 150 |
| 6 | $(CH_3)_3C\text{-}S\text{-}(CH_2)_2$ | 95 | 108 |
| 7 | $CH_2{=}C(CH_3)\text{-}CH_2$ | 95 | 57 |
| 8 | $Cl\text{-}(CH_2)_2$ | 95 | 85* |
| 9 | $Cl\text{-}CH[CH_2\text{-}S\text{-}C(CH_3)_3]_2$ | 95 | 100 |
| 10 | $Cl\text{-}(CH_2)_2\text{-}S\text{-}S\text{-}C(CH_3)_3$ | 72 | nd |
| 11 | $Cl\text{-}(CH_2)_3\text{-}S\text{-}S\text{-}C(CH_3)_3$ | 75 | nd |

(*) sous 0,2 mmHg  nd = non déterminée

EXEMPLES 12 à 14

Dans un tricol muni d'un réfrigérant, d'un thermomètre, d'une ampoule à brome et équipé d'une agitation magnétique, on introduit 0,1 mole d'alcool soufré ou de chlorure soufré. A 7 °C on a ajoute 0,22 mole d'$H_2O_2$ à 30%. On laisse revenir à température ordinaire et on agite jusqu'à l'obtention d'une solution homogène (environ 24 heures) puis on chauffe à reflux pendant 3 heures. Le mélange réactionnel est extrait au chloroforme et la phase organique est séchée sur du sulfate de sodium. Le chloroforme est éliminé sous pression réduite et l'on isole les composés souhaités.

Dans ces exemples l'alcool ou le chlorure soufré de formule $(CH_3)_3C\text{-}S\text{-}(CH_2)_nX$ réagit avec l'eau oxygénée pour former l'hydroxysulfone ou la chlorosulfone de formule $(CH_3)_3C\text{-}SO_2\text{-}(CH_2)_nX$. Le tableau III

ci-après indique le rendement (Rdt) de la réaction, exprimé en pourcentage, en fonction des valeurs de n et X.

## Tableau III

| Exemple | n | X | Rdt |
|---|---|---|---|
| 12 | 2 | Cl | 87 |
| 13 | 2 | OH | 75 |
| 14 | 3 | OH | 92 |

EXEMPLES 15 à 18

Dans un tricol muni d'un réfrigérant, d'un thermomètre, d'une entrée d'air et équipé d'une agitation magnétique, on introduit 50 millimoles d'acide méthacrylique, 60 millimoles d'alcool soufré, 60 ml de cyclohexane, de l'acide paratoluènesulfonique (35% molaire) et 800 ppm d'éther méthylique d'hydroquinone. On chauffe à reflux jusqu'à élimination de la quantité d'eau attendue. Le mélange réactionnel est alors lavé avec des solutions de soude, successivement 2N puis 5N. Après séchage de la phase organique, le cyclohexane est éliminé à l'aide d'un évaporateur rotatif pour donner le méthacrylate soufré attendu. Les alcools soufrés utilisés sont :

- pour l'exemple 15, l'alcool de l'exemple 2 ;
- pour l'exemple 16, l'alcool de l'exemple 11 ;
- pour l'exemple 17, l'alcool de l'exemple 1 ;
- pour l'exemple 18, l'alcool de l'exemple 4.

Le méthacrylate soufré est ensuite caractérisé par résonance magnétique nucléaire (RMN) :

- du proton au moyen d'un spectromètre JEOL PMX 60 Si, la référence étant le tétraméthylsilane.
- du carbone 13 au moyen d'un spectromètre BRUCKER WP 80, la référence étant le tétraméthylsilane.

EN RMN, dans ces exemples comme dans les exemples qui suivent, les positions cis, trans et gem des hydrogènes éthyléniques sont repérées par rapport à la fonction COO.

Le tableau IV ci-après rassemble d'une part le rendement (Rdt) de la réaction exprimé en pourcentage et d'autre part les déplacements chimiques, exprimés en ppm, observés dans les différents spectres.

Tableau IV

| Exemple | 15 | 16 | 17 | 18 |
|---|---|---|---|---|
| Rdt | 90 | 96 | 95 | 89 |
| RMN$^1$H | | | | |
| δ (H trans) | 5,55 (m, 1H) | 5,55 (m, 1H) | 5,50 (m, 1H) | 5,55 (m, 1H) |
| δ (H cis) | 6,10 (m, 1H) | 6,15 (m, 1H) | 6,10 (m, 1H) | 6,10 (m, 1H) |
| δ ($CH_2$-S)<br>δ ($CH_2$-O) | 4,20 (t, 2H)<br>2,55 (t, 2H) | 4,25 (t, 2H)<br>2,85 (t, 2H) | 4,15 (t, 2H)<br>2,55 (t, 2H) | 4,15 (t, 2H)<br>2,55 (t, 2H) |
| δ ($CH_3$) et δ ($CH_2$) | 1,95 (s, 3H)<br>1,60 (m, 4H)<br>1,40 (m, 4H)<br>1,30 (s, 9H) | 2,15 (m, 2H)<br>1,95 (m, 3H)<br>1,30 (s, 9H) | 2,00 (m, 3H)<br>1,60 (m, 6H)<br>1,35 (s, 9H) | 1,95 (m, 3H)<br>1,35 (m, 18H)<br>1,35 (s, 9H) |
| RMN$^{13}$C | | | | |
| δ (CO) | 166,8 | 166,9 | 166,9 | 167,2 |
| δ ($CH_2$=) | 124,5 | 125,0 | 124,7 | 124,8 |
| δ (C=) | 136,0 | 136,0 | 136,1 | 136,4 |
| δ (O-$CH_2$) | 64,1 | 62,8 | 64,2 | 64,9 |
| δ ($CH_2$-S) | 25,1 | 36,7 | 25,2 | - |
| δ ($CH_2$) | 27,6-29,2 | 28,2 | 27,7-29,1 | - |
| δ (C) | 41,1 | 47,3 | 41,3 | 41,4 |
| δ ($CH_3$) | 30,5<br>17,8 | 29,7<br>18,1 | 30,7<br>17,9 | 30,8<br>18,0 |

EXEMPLE 19

La procédure expérimentale de l'exemple 15 est répétée en remplaçant l'acide méthacrylique par une quantité équivalente d'acide acrylique. L'acrylate soufré correspondant est ainsi obtenu avec un rendement de 92%.

EXEMPLES 20 à 24

Dans un réacteur on place x mole de méthacrylate de potassium dilué dans y moles d'eau. On introduit ensuite dans le réacteur 0,1 mole de chlorure soufré de formule $(CH_3)_3C$-S-(-$CH_2)_6Cl$, 0,1 x mole de chlorure de tricaprylylméthylammonium (commercialisé par la société FLUKA sous la dénomination ALIQUAT® 336) et 50 ppm (par rapport au méthacrylate de potassium) d'éther méthylique d'hydroquinone. Ce mélange est alors chauffé sous agitation à la température T (exprimée en degrés Celsius) pendant la durée t (exprimée en heures). La phase organique est alors recueillie, éventuellement par extraction au dichlorométhane, lavée à l'eau puis séchée. Dans le cas d'une extraction, le méthacrylate de formule $CH_2$ = C-$(CH_3)$-$CO_2$-$(CH_2)_6$-S-$C(CH_3)_3$ est isolé par évaporation du dichlorométhane. Le tableau V ci-après indique, en fonction des valeurs de x, y, T et t, le rendement (Rdt) obtenu en méthacrylate, exprimé en pourcentage. L'exemple 23 est donné à titre comparatif afin d'illustrer l'effet défavorable d'une température réactionnelle trop basse.

Tableau V

| Exemple | 20 | 21 | 22 | 23 | 24 |
|---|---|---|---|---|---|
| x | 0,11 | 0,15 | 0,2 | 0,2 | 0,15 |
| y | 0,55 | 0,75 | 1,0 | 1,0 | 1,5 |
| T | 100 | 100 | 80 | 38 | 100 |
| t | 5 | 5 | 18 | 18 | 5 |
| Rdt | 69 | 83 | 75 | 5 | 77 |

EXEMPLES 25 à 28

On reproduit le mode opératoire des exemples 20 à 24 en utilisant comme chlorure soufré (0,1 mole) celui de formule $[(CH_3)_3C$-S-$CH_2]_2$ CHCl et en utilisant systématiquement 0,2 mole de méthacrylate de potassium, 1 mole d'eau et 0,02 mole d'agent de transfert de phase (celui-ci est le chlorure de tricaprylylméthylammonium pour les exemples 25 à 27, le chlorure de tétraméthylammonium pour l'exemple 28). Le tableau VI ci-après indique, en fonction des valeurs de T et t, le rendement (Rdt) observé en méthacrylate de formule $CH_2$ = $C(CH_3)$-$CO_2$-CH[$CH_2$-S-$C(CH_3)_3]_2$, exprimé en pourcentage.

Tableau VI

| Exemple | 25 | 26 | 27 | 28 |
|---|---|---|---|---|
| T | 100 | 100 | 100 | 80 |
| t | 0,5 | 1 | 5 | 18 |
| Rdt | 25 | 70 | 76 | 75 |

EXEMPLES 29 à 45

Dans un réacteur on place 0,15 mole de méthacrylate de potassium dilué dans 0,75 mole d'eau. On introduit ensuite dans le réacteur 0,1 mole de chlorure soufré, 0,01 mole de chlorure de tricaprylylméthylammonium et 50 ppm (par rapport au méthacrylate de potassium) d'éther méthylique d'hydroquinone. Le mélange est alors chauffé à 98 °C sous agitation pendant 5 heures. La phase organique est alors traitée selon le mode opératoire des exemples 20 à 24. Le rendement en méthacrylate soufré (Rdt), exprimé en pourcentage, est indiqué au tableau VII en fonction de la nature du chlorure soufré de départ (formule RCl).

Le cas échéant, le tableau indique également la température d'ébullition T (exprimée en degrés Celsius) du méthacrylate soufré ainsi que les caractéristiques des spectres de résonance magnétique nucléaire (RMN) du proton et du carbone 13, obtenus comme aux exemples 15 à 18, pour les composés nouveaux.

Tableau VII

| Exemple | 29 | 30 | 31 |
|---|---|---|---|
| R | $(CH_2)_2SC(CH_3)_3$ | $(CH_2)_3SC(CH_3)_3$ | $(CH_2)_2S(CH_2)_2SC(CH_3)_3$ |
| Rdt | 95 | 92 | 50 |
| T°C | nd | 105 (1 mmHg) | nd |
| **Exemple** | **32** | **33** | **34** |
| R | $(CH_2)_2SCH_2C_6H_5$ | $(CH_2)_6SC(CH_3)_3$ | $CH[CH_2SC(CH_3)_3]_2$ |
| Rdt | 82 | 90 | 80 |
| **Exemple** | **35** | **36** | **37** |
| R | $(CH_2)_3S_2C(CH_3)_3$ | $(CH_2)_2SCH_2CH(CH_3)_2$ | $(CH_2)_2S(CH_2)_3CH_3$ |
| Rdt | 80 | 90 | 87 |
| **Exemple** | **38** | **39** | **40** |
| R | $CH_2S(CH_2)_3CH_3$ | $(CH_2)_2S(CH_2)_9CH_3$ | $(CH_2)_2$S-dicyclopentadiényl |
| Rdt | 55 | 75 | 68 |
| T°C | 82 (2 mmHg) | 145 (1,5 mmHg) | nd |

nd = non déterminé.

Tableau VII ( suite)

| Exemple | 41 | 42 | 43 |
|---|---|---|---|
| R | $(CH_2)_2$S-cyclohexyl | $(CH_2)_2SCH_2$-thiényl-3 | $(CH_2)_2SCH_2$ 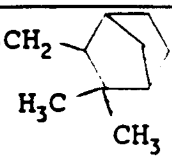 |
| Rdt | 92 | 60 | 85 |
| T°C | 142 (1 mmHg) | nd | nd |
| RMN[1]H | | | |
| δ (H trans) | 5,60(m, 1H) | 5,55(m, 1H) | 5,55(m, 1H) |
| δ (H cis) | 6,15(m, 1H) | 6,05(m, 1H) | 6,15(m, 1H) |
| δ (arom) | | 7,1 (m, 3H) | |
| δ ($CH_2$-O) | 4,30(t, 2H) | 4,2 (t, 2H) | 4,3 (t, 2H) |
| et ($CH_2$-S) | 2,75(t, 2H) | 3,75(s, 2H) | 2,75(t, 2H) |
| | | 2,65(t, 2H) | 2,65(s, 2H) |
| δ ($CH_3$) | 1,95(s, 3H) | 1,95(S, 3H) | 1,95(m, 3H) |
| et ($CH_2$) | 1,0-2,0(m, 11H) | | 1,1-1,9(m, 9H) |
| et (CH) | | | 0,97(s, 3H) |
| | | | 0,85(s, 3H) |

Tableau VII (fin)

| Exemple | 44 | 45 |
|---|---|---|
| R | $(CH_2)_2S(CH_2)_2-OCO-C(CH_3)=CH_2$ | $(CH_2)_2SCH_2-C(CH_3)=CH_2$ |
| Rdt | 80 | 85 |
| T°C | nd | 90 (1 mmHg) |
| RMN[1]H | | |
| δ (H trans) | 5,65(m, 2H) | 5,60(m, 1H) |
| δ (H cis) | 6,15(m, 2H) | 6,15(m, 1H) |
| δ ($CH_2$=) | | 4,85(m, 2H) |
| δ ($CH_2$-O) | 4,35(t, 4H) | 4,30(t, 2H) |
| & ($CH_2$-S) | 2,90(t, 4H) | 3,20(s, 2H) |
| | | 2,70(t, 2H) |
| δ ($CH_3$) | 2,0(s, 6H) | 1,95(S, 3H) |
| | | 1,80(s, 3H) |
| RMN[13]C | nd | |
| δ (CO) | | 166,8 |
| δ ($CH_2$=) | | 125,4-113,7 |
| δ (C=) | | 140,7-136,0 |
| δ ($CH_2$-O) | | 63,1 |
| δ ($CH_2$-S) | | 39,2-29,0 |
| δ ($CH_3$) | | 20,2-18,0 |

**nd = non déterminé**

EXEMPLES 46 à 47

Dans un tricol muni d'un réfrigérant, d'un thermomètre et équipé d'une agitation magnétique, on introduit x mole de méthacrylate de potassium dilué dans 5x moles d'eau. On ajoute ensuite 0,1 mole de chlorure soufré dilué dans 25 ml de chloroforme et 0,1x mole de chlorure de tricaprylylméthylammonium. Le mélange réactionnel est alors chauffé pendant 18 heures à 60 ° C. A la fin de la réaction le mélange est décanté. La phase organique est recueillie puis lavée et séchée. Après évaporation du chloroforme à l'aide d'un évaporateur rotatif on recueille un liquide marron. Le tableau VIII ci-après indique le rendement (Rdt) de la réaction en méthacrylate soufré, exprimé en pourcentage molaire par rapport au chloroforme soufré, en fonction de la valeur de x et de la nature du chlorure.

## Tableau VIII

| Exemple | Chlorure | x | Rdt |
|---|---|---|---|
| 46 | $[(CH_3)_3CSCH_2]_2CHCl$ | 0,2 | 70 |
| 47 | $(CH_3)_3CS(CH_2)_6Cl$ | 0,15 | 85 |

EXEMPLES 48 à 56

Dans un réacteur on place 0,12 mole de méthacrylate de potassium dilué dans 25 ml d'eau. On introduit ensuite dans le réacteur 0,1 mole de chlorure soufré RCl dilué dans 25 ml de chloroforme, 0,01 mole d'hydrogénosulfate de tétrabutylammonium comme agent de transfert de phase et 50 ppm (par rapport au méthacrylate) d'éther méthylique d'hydroquinone. Le mélange est alors chauffé à reflux sous agitation pendant 18 heures. Le méthacrylate soufré est ensuite isolé comme indiqué à l'exemple 46. Le tableau IX ci-après indique le rendement (Rdt) de la réaction en méthacrylate soufré, exprimé en pourcentage molaire par rapport au chlorure soufré, en fonction de la valeur de R.

## Tableau IX

| Exemple | 48 | 49 | 50 |
|---|---|---|---|
| R | $(CH_2)_2SC(CH_3)_3$ | $(CH_2)_3S_2C(CH_3)_3$ | $(CH_2)_2SCH_2CH(CH_3)_2$ |
| Rdt | 90 | 80 | 90 |
| **Exemple** | **51** | **52** | **53** |
| R | $(CH_2)_2$S-cyclohexyl | $(CH_2)_2SCH_2$-thiényl-3 | $CH_2S(CH_2)_3CH_3$ |
| Rdt | 92 | 60 | 55 |
| **Exemple** | **54** | **55** | **56** |
| R | $(CH_2)_2S(CH_2)_9CH_3$ | $(CH_2)_2SCH_2$- 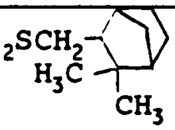 | $(CH_2)_2SCH_2C(CH_3)=CH_2$ |
| Rdt | 75 | 85 | 85 |

EXEMPLES 57 à 58

On reproduit le mode opératoire des exemples 48 à 56 en remplaçant le méthacrylate de potassium par l'acrylate de potassium. Le tableau X ci-après indique le rendement de la réaction en acrylate soufré en fonction de la nature de R.

Tableau X

| Exemple | 57 | 58 |
|---|---|---|
| R | $(CH_2)_2SC(CH_3)_3$ | $(CH_2)_2SCH_2C(CH_3)=CH_2$ |
| Rdt | 50 | 35 |

EXEMPLES 59 à 63

Dans un tricol muni d'un réfrigérant, d'un thermomètre et équipé d'une agitation magnétique, on introduit 0,2 mole de méthacrylate de potassium. On ajoute ensuite 0,1 mole de chlorure soufré - $(CH_3)_3CS-(CH_2)_6Cl$ pour l'exemple 59, $[(CH_3)_3CSCH_2]_2CHCl$ pour les exemples 60 à 63 - dilué dans 30 ml de solvant et 0,02 mole d'agent de transfert de phase. Le mélange réactionnel est alors chauffé à la température T (exprimée en degrés Celsius) pendant 18 heures. A la fin de la réaction, le mélange est filtré. Le filtrat est traité dans un évaporateur rotatif afin d'éliminer le solvant. On recueille un liquide marron. Le tableau XI ci-après indique le rendement (Rdt) de la réaction en méthacrylate soufré, exprimé en pourcentage molaire par rapport au chlorure soufré, en fonction de T et de la nature du solvant et de l'agent de transfert de phase. L'exemple 63 est comparatif en raison de la température trop élevée de reflux du solvant choisi.

Tableau XI

| Exemple | 59 | 60 | 61 | 62 | 63 |
|---|---|---|---|---|---|
| Solvant | acéto-nitrile | acéto-nitrile | acéto-nitrile | acéto-nitrile | toluène |
| Agent de transfert de phase | Aliquat® 336 | poly-éthylène glycol | éther-couronne | Aliquat® 336 | éther-couronne |
| T | 80 | 80 | 80 | 80 | 110 |
| Rdt | 80 | 80 | 90 | 60 | 0 |

EXEMPLE 64

Dans un réacteur on place 0,15 mole de méthacrylate de potassium puis on introduit 0,1 mole de chlorure de (phénylthio)méthyle dilué dans 30 ml d'acétonitrile, 0,01 mole de chlorure de tricaprylylméthylammonium et 50 ppm (par rapport au méthacrylate) d'éther méthylique d'hydroquinone. Ce mélange est alors chauffé à reflux sous agitation pendant 5 heures. A la fin de la réaction le mélange est filtré puis, par

évaporation de l'acétonitrile, on recueille avec un rendement de 65% le méthacrylate de (phénylthio)-méthyle, dont la température d'ébullition sous 1,7 mmHg est égale à 115 °C.

EXEMPLES 65 à 69

Dans un réacteur on place 0,1 mole de sulfure (méth)acrylique. Dans ce réacteur maintenu à 0 °C on introduit goutte à goutte 0,1 mole d'eau oxygénée à 30%. Lorsque l'addition est terminée, le mélange est agité à 18 °C pendant 24 heures. Le mélange obtenu est alors extrait au chloroforme. Après séchage de la phase organique, le composé final est obtenu par évaporation du solvant. Le tableau XII ci-après indique le rendement de la réaction en sulfoxyde (méth)acrylique, exprimé en pourcentage par rapport au sulfure (méth)acrylique de départ, en fonction de la nature de celui-ci (identifié par le numéro de l'exemple précédent correspondant), ainsi que les données des spectres RMN.

Tableau XII

| Exemple | 65 | 66 | 67 | 68 | 69 |
|---|---|---|---|---|---|
| sulfure | ex.29 | ex.57 | ex.30 | ex.17 | ex.18 |
| Rdt | 90 | 91 | 96 | 98 | 97 |
| RMN$^1$H | | | | | |
| δ (H trans) | 5,6<br>(m, 1H) | 5,9<br>(m, 1H) | 5,55<br>(m, 1H) | 5,55<br>(m, 1H) | 5,55<br>(m, 1H) |
| δ (H cis) | 6,15<br>(m, 1H) | 6,1-6,45<br>(m, 2H) | 6,1<br>(m, 1H) | 6,1<br>(m, 1H) | 6,1<br>(m, 1H) |
| δ ($CH_2$-O) | 4,65<br>(m, 1H) | 4,65<br>(m, 2H) | 4,35<br>(m, 2H) | 4,15<br>(t, 2H) | 4,15<br>(t, 2H) |
| & ($CH_2$-S) | 4,55<br>(m, 1H)<br>2,9<br>(m, 1H)<br>2,7<br>(m, 1H) | 2,9<br>(m, 2H) | 2,6<br>(m, 2H) | 2,45<br>(t, 2H) | 2,55<br>(t, 2H) |
| δ ($CH_3$)<br>et δ ($CH_2$) | 1,95<br>(m, 3H)<br>1,3<br>(s, 9H) | 1,35<br>(s, 9H) | 2,2<br>(m, 2H)<br>1,95<br>(m, 3H)<br>1,3<br>(s, 9H) | 2,0<br>(m, 3H)<br>1,9<br>(m, 6H)<br>1,3<br>(s, 9H) | 1,95<br>(m, 3H)<br>1,35<br>(m,18H)<br>1,35<br>(s, 9H) |
| RMN$^{13}$C | | | nd | | |
| δ (CO) | 166,6 | 165,3 | | 166,9 | 167,3 |
| δ ($CH_2$=) | 125,9 | 131,2 | | 124,8 | 124,8 |
| δ (C=) | 135,5 | 127,6 | | 135,9 | 136,4 |
| δ ($CH_2$-O) | 58,2 | 58,1 | | 63,8 | 64,5 |
| δ ($CH_2$-S) | 45,1 | 45,7 | | 45,0 | |
| δ ($CH_2$) | | | | 27,9-23,0 | |
| δ (C-C) et | 52,8-22,4 | 53,6-22,4 | | 52,3-22,4 | |
| δ ($CH_3$) | 17,8 | | | 17,8 | 18,1 |

nd = non déterminé

EXEMPLE 70

On applique au sulfure acrylique $CH_2=CH-CO_2-(CH_2)_3SC(CH_3)_3$ - obtenu selon l'enseignement de la littérature - le mode opératoire des exemples 64 à 69. On obtient avec un rendement de 95% le sulfoxyde correspondant, présentant les caractéristiques de RMN du proton suivantes :

δ (H trans) = 5,9 (m, 1H) δ (H cis) = 6,3 (m, 1H)

δ (H gem) = 6,15 (m, 1H)
δ ($CH_2$-O & $CH_2$-S) = 4,35 (t, 2H) et 2,55 (t, 2H)
δ ($CH_3$) et δ ($CH_2$) = 2,35 (m, 2H) et 1,55 (s, 9H) et 1,35 (s, 9H)

EXEMPLE 71

On applique au sulfure méthacrylique de l'exemple 45 le mode opératoire des exemples 64 à 69. On obtient avec un rendement de 99% le sulfoxyde correspondant, présentant les caractéristiques de RMN du proton suivantes :
δ (H trans) = 5,6 (m, 1H) δ (H cis) = 6,1 (m, 1H)
δ ($CH_2$) = 5,1 (m, 2H) δ ($CH_3$) = 1,95 (m, 6H)
δ ($CH_2$-O, $CH_2$-S) = 4,6 (t, 2H), 3,5 (s, 2H), 3,1 (m, 2H)
Le spectre de RMN du carbone 13 présente les données suivantes :
δ (CO) = 166,5 δ ($CH_2$=) = 126,0 et 118,1
δ (C=) = 135,4 et 135,1 δ ($CH_2$-O) = 62,3
δ ($CH_2$-S) = 57,2 et 51,7 δ ($CH_3$) = 22,6 et 17,8

EXEMPLES 72 à 74

Dans un réacteur on place 0,1 mole de sulfure (méth)acrylique. Dans ce réacteur maintenu à 7°C on introduit goutte à goutte 0,22 mole d'eau oxygénée à 30%. Lorsque l'addition est terminée, le mélange est agité à 18°C pendant 24 heures puis chauffé à 45°C pendant 72 heures. Le mélange obtenu est extrait au chloroforme. Après séchage de la phase organique, le composé final est obtenu par évaporation du solvant. Le tableau XIII ci-après indique le rendement de la réaction en sulfone (méth)acrylique, exprimé en pourcentage par rapport au sulfure de départ, en fonction de la nature de celui-ci (identifié par le numéro de l'exemple précédent où sa synthèse a été décrite), ainsi que les caractéristiques des spectres de RMN du proton et du carbone 13.

TABLEAU XIII

| Exemple | 72 | 73 | 74 |
|---|---|---|---|
| Sulfure | ex.29 | ex.18 | ex.45 |
| Rdt | 98 | 95 | 99 |
| RMN $^1H$ | | | |
| δ (H trans) | 5,65 (m,1H) | 5,55 (m,1H) | 5,60 (m,1H) |
| δ (H cis) | 6,15 (m,1H) | 6,10 (m,1H) | 6,15 (m,1H) |
| δ ($CH_2$=) | | | 5,15 (m,2H) |
| δ ($CH_2$-O) | 4,65 (t,2H) | 4,15 (t,2H) | 4,60 (t,2H) |
| & ($CH_2$-S) | 3,35 (t,2H) | 2,85 (t,2H) | 3,75 (s,2H)<br>3,45 (m,2H) |
| δ ($CH_3$) | 2,0 (m,3H)<br>1,3 (s,9H) | 1,95 (m,3H)<br>1,35 (m,18H)<br>1,35 (s,9H) | 2,0 (m,6H) |

TABLEAU XIII (suite)

| Exemple | 72 | 74 |
|---|---|---|
| RMN $^{13}C$ | | |
| δ (CO) | 166,7 | 166,4 |
| δ ($CH_2$=) | 126,4 | 126,4-120,6 |
| δ (C=) | 135,4 | 135,4-133,5 |
| δ ($CH_2$-O) | 56,9 | 62,2 |
| δ ($CH_2$-S) | 45,1 | 57,6-50,3 |
| δ ($CH_3$) et δ (C-C) | 59,5-23,0-17,8 | 22,5-17,9 |

EXEMPLE 75

Dans un tricol muni d'un réfrigérant, d'un thermomètre, d'une ampoule à brome et équipé d'une agitation magnétique, on introduit 1 mole de soude dans 400 ml d'éthanol. A 50°C on ajoute 0,9 mole de mercaptoéthanol puis on poursuit le chauffage à 50°C durant 30 mn. Après retour à la température ambiante on additionne goutte à goutte 0,9 mole de bromopropionate d'éthyle. A la fin de l'addition le mélange est porté au reflux pendant 6 heures. Après refroidissement le sel formé est éliminé par filtration. Le filtrat est neutralisé par HCl 1 N puis la phase organique est extraite au dichlorométhane. Les phases organiques sont lavées puis séchées.

Après élimination du dichlorométhane sous pression réduite, on isole un liquide dont la température d'ébullition sous 1 mmHg est égale à 140°C. Le rendement en composé de formule $HO(CH_2)_2S(CH_2)$-

$_3CO_2C_2H_5$ est de 76%.

Dans un tricol muni d'un réfrigérant, d'un thermomètre, d'une ampoule à brome et équipé d'une agitation magnétique, on introduit 0,04 mole de l'alcool soufré, préparé précédemment, dans 20 ml de chloroforme sec. On ajoute goutte à goutte 0,04 mole de chlorure de thionyle dilué dans 10 ml de chloroforme. A mi-addition, le mélange est porté à reflux et le chauffage est poursuivi pendant 4 heures. Après élimination du chloroforme sous pression réduite, le chlorure soufré de formule $Cl(CH_2)_2S(CH_2)_3CO_2C_2H_5$, obtenu avec un rendement de 99%, est purifié par distillation. Sa température d'ébullition sous 1 mm Hg est de 130 °C.

Dans un tricol muni d'un réfrigérant, d'un thermomètre, d'une ampoule à brome et équipé d'une agitation magnétique, on introduit 0,1 mole de méthacrylate de potassium dilué dans 9 ml d'eau. On ajoute alors 0,07 mole de chlorure soufré, préparé précédemment, dans 50 ml de chloroforme puis on introduit 0,01 mole d'hydrogénosulfate de tétra-n-butylammonium. Le mélange est alors chauffé pendant 20 heures à 60 °C. La phase organique est recueillie, lavée puis séchée. Après élimination du chloroforme le méthacrylate de 2-[(3-éthoxycarbonylpropyl)thio] éthyle, obtenu avec un rendement de 65%, est purifié par chromatographie sur colonne. Les caractéristiques de ses spectres de RMN du proton et du carbone 13 figurent au tableau XIV ci-après.

**Revendications**

**1.** Procédé de préparation d'un (méth)acrylate de formule générale :

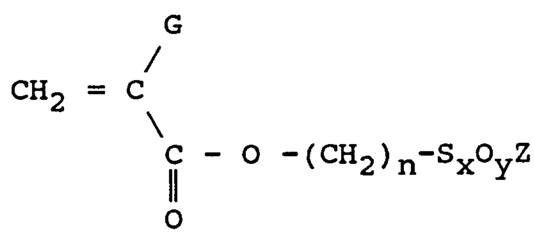

(IX)

dans laquelle :

- G désigne un atome d'hydrogène ou un radical méthyle ;
- n est un nombre entier compris entre 1 et 12 inclusivement,
- x vaut 1 ou 2,
- y vaut 0, 1 ou 2, y ne pouvant représenter 1 ou 2 que lorsque x = 1, et
- Z est un radical alkyle, cycloalkyle, alcényle, aryle, alkylaryle, arylalkyle ou $\beta$-thiénylméthyle, Z étant porteur le cas échéant d'une fonction ester,

à partir d'un (méth)acrylate de formule générale :

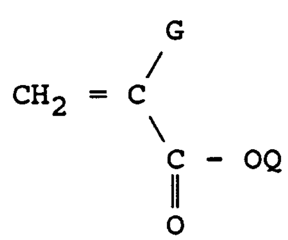

(VII)

dans laquelle G est tel que défini ci-dessus et Q est un métal alcalin,

par réaction dudit (méth)acrylate de formule (VII) avec un composé soufré de formule $ZS_x(CH_2)_nX$ dans laquelle x, n et Z ont les significations indiquées précédemment et X est un atome d'halogène, ladite réaction étant effectuée soit en milieu au moins partiellement aqueux à une température de 50 °C à 100 °C soit dans un solvant organique dont la température d'ébullition ne dépasse pas 100 °C, en présence d'au moins un inhibiteur de polymérisation et d'au moins un agent de transfert de phase et conduisant à un (méth)acrylate de formule (VI-a) dans laquelle y = 0 et x = 1 ou 2, auquel cas, si x = 1, cette première réaction peut être suivie par une réaction d'oxydation au moyen d'au moins un agent

oxydant à une température généralement comprise entre 10°C et 60°C environ afin d'obtenir un (méth)acrylate de formule (VI) dans laquelle y vaut 1 ou 2.

**2.** Procédé selon la revendication 1, caractérisé en ce que la réaction entre le (méth)acrylate de formule (VII) et le composé soufré est effectuée en utilisant l'eau comme solvant, à une température comprise entre 70°C et 100°C.

**3.** Procédé selon l'une des revendications 1 et 2, caractérisé en ce que l'agent de transfert de phase est choisi parmi les sels d'ammonium, de phosphonium et d'arsénium quaternaires, les éthers de polyéthylène glycols, les aminoéthers et les complexants macrohétérocycliques.

**4.** Procédé selon l'une des revendications 1 à 3, caractérisé en ce que l'agent de transfert de phase est utilisé à raison de 5 à 30% en moles par rapport au (méth)acrylate de formule (VII).

**5.** Procédé selon l'une des revendications 1 à 4, caractérisé en ce que le rapport molaire

$$\frac{\text{méthacrylate (VII)}}{\text{composé soufré}}$$

est compris entre 1 et 1,6.

**6.** Procédé selon l'une des revendications 1 à 5, caractérisé en ce que la durée de la réaction entre le (méth)acrylate de formule (VII) et le composé soufré est comprise entre 30 minutes et 20 heures.

**7.** Procédé selon l'une des revendications 1 à 6, caractérisé en ce que l'agent oxydant est choisi parmi l'eau oxygénée, les peracides organiques, l'oxygène, l'air et le diméthylsulfoxyde.

**8.** Procédé selon l'une des revendications 1 à 7, caractérisé en ce que l'agent oxydant est utilisé dans un rapport molaire

$$\frac{\text{agent oxydant}}{\text{(méth)acrylate (VI-a)}}$$

au moins égal à 1 pour obtenir un sulfoxyde (y = 1), au moins égal à 2 pour obtenir une sulfone (y = 2).

**9.** Procédé selon l'une des revendications 1 à 8, caractérisé en ce que la durée de la réaction d'oxydation est comprise entre 3 et 90 heures.

**Claims**

**1.** Process for the preparation of a (meth)acrylate of general formula:

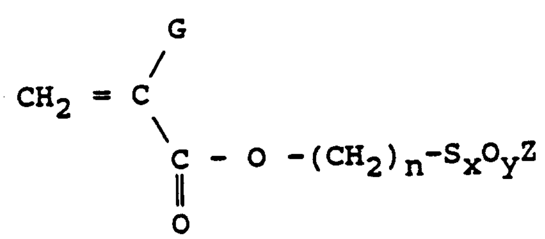

(IX)

in which:

- G denotes a hydrogen atom or a methyl radical;
- n is an integer between 1 and 12 inclusive,
- x is equal to 1 or 2,
- y is equal to 0, 1 or 2, it only being possible for y to represent 1 or 2 when x = 1, and
- Z is an alkyl, cycloalkyl, alkenyl, aryl, alkylaryl, arylalkyl or β-thienylmethyl radical, Z carrying, if necessary, an ester function,

from a (meth)acrylate of general formula:

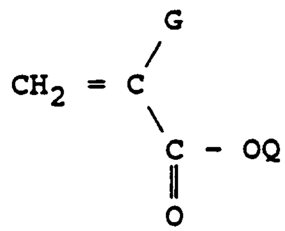

(VII)

in which G is as defined above and Q is an alkali metal, by reaction of the said (meth)acrylate of formula (VII) with a sulphur-containing compound of formula $ZS_x(CH_2)_nX$ in which x, n and Z have the meanings indicated above and X is a halogen atom, the said reaction being carried out either in an at least partially aqueous medium at a temperature of 50 °C to 100 °C or in an organic solvent, the boiling point of which does not exceed 100 °C, in the presence of at least one polymerization inhibitor and at least one phase transfer agent and leading to a (meth)acrylate of formula (VI-a) in which y = 0 and x = 1 or 2, in which case, if x = 1, this first reaction may be followed by an oxidation reaction by means of at least one oxidizing agent at a temperature of generally between 10 °C and 60 °C approximately in order to obtain a (meth)acrylate of formula (VI) in which y is equal to 1 or 2.

**2.** Process according to Claim 1, characterized in that the reaction between the (meth)acrylate of formula (VII) and the sulphur-containing compound is carried out using water as solvent, at a temperature of between 70 °C and 100 °C.

**3.** Process according to one of Claims 1 and 2, characterized in that the phase transfer agent is chosen from quaternary ammonium, quaternary phosphonium and quaternary arsenium salts, polyethylene glycol ethers, aminoethers and macroheterocyclic complexing agents.

**4.** Process according to one of Claims 1 to 3, characterized in that the phase transfer agent is used in an amount of from 5 to 30 mol% relative to the (meth)acrylate of formula (VII).

**5.** Process according to one of Claims 1 to 4, characterized in that the molar ratio

$$\frac{\text{(meth)acrylate (VII)}}{\text{sulphur-containing compound}}$$

is between 1 and 1.6.

**6.** Process according to one of Claims 1 to 5, characterized in that the duration of the reaction between the (meth)acrylate of formula (VII) and the sulphur-containing compound is between 30 minutes and 20 hours.

**7.** Process according to one of Claims 1 to 6, characterized in that the oxidizing agent is chosen from hydrogen peroxide, organic peracids, oxygen, air and dimethyl sulphoxide.

**8.** Process according to one of Claims 1 to 7, characterized in that the oxidizing agent is used in a molar ratio

oxidizing agent

(meth)acrylate (VI-a)

of at least 1 in order to obtain a sulphoxide (y = 1), and of at least 2 in order to obtain a sulphone (y = 2).

**9.** Process according to one of Claims 1 to 8, characterized in that the duration of the oxidation reaction is between 3 and 90 hours.

**Patentansprüche**

**1.** Verfahren zur Herstellung eines Methacrylats der allgemeinen Formel

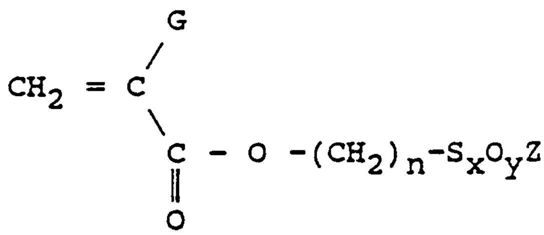

(IX),

worin bedeuten:

- G ein Wasserstoffatom oder eine Methylgruppe,
- n eine ganze Zahl von 1 bis 12,
- x 1 oder 2,
- y 0, 1 oder 2, wobei y nicht 1 oder 2 sein kann, wenn x 1 ist, und
- Z Alkyl, Cycloalkyl, Alkenyl, Aryl, Alkylaryl, Arylalkyl oder β-Thienylmethyl, wobei Z gegebenenfalls eine Estergruppe trägt,

aus einem Methacrylat der allgemeinen Formel

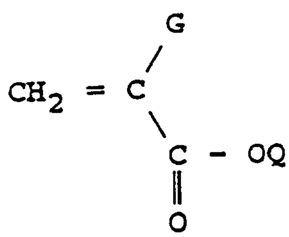

(VII),

worin G das gleiche wie oben bedeutet und Q ein Alkalimetall ist, durch Umsetzung des Methacrylats der Formel (VII) mit einer schwefelhaltigen Verbindung der Formel $ZS_x(CH_2)_nX$, worin x, n und Z die oben angegebenen Bedeutungen haben und X ein Halogenatom ist, wobei die Umsetzung entweder in einem zumindest teilweise wäßrigen Medium bei einer Temperatur von 50 bis 100 °C oder in einem organischen Lösungsmittel, dessen Siedetemperatur höchstens 100 °C beträgt, in Gegenwart von mindestens einem Polymerisationsinhibitor und einem oder mehreren Phasentransfermitteln durchgeführt wird und zu einem Methacrylat der Formel (VI-a) führt, in der y gleich 0 und x gleich 1 oder 2 sind, wobei dann, wenn x gleich 1 ist, auf diese erste Reaktion eine Oxidationsreaktion mit einem oder mehreren Oxidationsmitteln bei einer Temperatur, die üblicherweise etwa 10 bis 60 °C beträgt, folgen kann, um ein Methacrylat der Formel (VI) zu erhalten, worin y gleich 1 oder 2 ist.

**2.** Verfahren nach Anspruch 1, dadurch gekennzeichnet, daß die Umsetzung des Methacrylats der Formel (VII) mit der schwefelhaltigen Verbindung unter Verwendung von Wasser als Lösungsmittel bei einer Temperatur von 70 bis 100 °C durchgeführt wird.

3. Verfahren nach einem der Ansprüche 1 und 2, dadurch gekennzeichnet, daß das Phasentransfermittel ausgewählt ist unter quaternären Ammonium-, Phosphonium- und Arsoniumsalzen, Polyethylenglykolethern, Aminoethern und makroheterocyclischen Komplexierungsmitteln.

4. Verfahren nach einem der Ansprüche 1 bis 3, dadurch gekennzeichnet, daß das Phasentransfermittel in einem Anteil von 5 bis 30 Mol-%, bezogen auf das Methacrylat der Formel (VII), verwendet wird.

5. Verfahren nach einem der Ansprüche 1 bis 4, dadurch gekennzeichnet, daß das Molverhältnis Methacrylat (VII)/schwefelhaltige Verbindung 1 bis 1,6 beträgt.

6. Verfahren nach einem der Ansprüche 1 bis 5, dadurch gekennzeichnet, daß die Umsetzung des Methacrylats der Formel (VII) mit der schwefelhaltigen Verbindung 30 min bis 20 h dauert.

7. Verfahren nach einem der Ansprüche 1 bis 6, dadurch gekennzeichnet, daß das Oxidationsmittel unter Wasserstoffperoxid, organischen Persäuren, Sauerstoff, Luft und Dimethylsulfoxid ausgewählt wird.

8. Verfahren nach einem der Ansprüche 1 bis 7, dadurch gekennzeichnet, daß das Oxidationsmittel in einem Molverhältnis Oxidationsmittel/Methacrylat (VI-a) von mindestens 1 verwendet wird, um ein Sulfoxid ($y = 1$) zu erhalten und in einem Molverhältnis von mindestens 2 verwendet wird, um ein Sulfon ($y = 2$) zu erhalten.

9. Verfahren nach einem der Ansprüche 1 bis 8, dadurch gekennzeichnet, daß die Oxidationsreaktion 3 bis 90 h dauert.